Europäisches Patentamt

European Patent Office

Office européen des brevets

(11) Publication number: **0 037 043**
**B1**

(12) **EUROPEAN PATENT SPECIFICATION**

(45) Date of publication of patent specification: **21.11.84**

(21) Application number: **81102132.8**

(22) Date of filing: **20.03.81**

(51) Int. Cl.³: **A 61 K 9/06,** A 61 K 37/02, A 61 K 45/06 // (A61K37/02, 31/70, 31/505, 31/305, 31/045)

(54) Stabilised ophthalmic formulation.

(30) Priority: **21.03.80 GB 8009589**

(43) Date of publication of application:
**07.10.81 Bulletin 81/40**

(45) Publication of the grant of the patent:
**21.11.84 Bulletin 84/47**

(84) Designated Contracting States:
**BE CH DE FR GB LI LU NL SE**

(56) References cited:
FR-A-2 255 909
GB-A-1 340 518
US-A-2 703 777

UNLISTED DRUGS, vol. 2, February 1977.
Chatham, New York, US

CHEMICAL ABSTRACTS, vol. 55, nr. 21,
October 16, 1961, abstract 21480b Columbus,
Ohio, US MICHAEL IANNARONE: "Ophthalmic
solutions in pharmacy"

(73) Proprietor: **THE WELLCOME FOUNDATION LIMITED**
**183-193 Euston Road**
**London NW1 2BP (GB)**

(72) Inventor: **Marsden, Peter Hugh**
**24 Shepherds Lane**
**Dartford Kent (GB)**
Inventor: **Reader, Michael John**
**31 St. Johns Way Borstal**
**Rochester Kent (GB)**

(74) Representative: **Berg, Wilhelm, Dr. et al**
**Patentanwälte Dr. Berg Dipl.-Ing. Stapf Dipl.-Ing.**
**Schwabe Dr. Dr. Sandmair Postfach 86 02 45**
**Stuntzstrasse 16**
**D-8000 München 86 (DE)**

(56) References cited:
UNLISTED DRUGS, vol. 26, October 1974,
Chatham, New York, US

Martindale: The Extra Pharmacopoeia

The Pharmaceutical Codex (1979)

**0 037 043**

### Description

The present invention relates to isotonic liquid pharmaceutical formulations, particularly to isotonic aqueous opthalmic formulations comprising trimethoprim and polymyxim, a vehicle therefore, a mercury-containing preservative and an isotonic agent.

Thiomersal, sodium ethylmercurithiosalicylate, is an effective antifungal and antibacterial agent widely used as a preservative in pharmaceutical formulations, particularly liquid formulations such as ophthalmic solutions. It has long been recognised that Thiomersal decomposes in aqueous media but as its biological, i.e. preservative, action is not impaired thereby such decomposition has not, in the past, been of great concern. However, in more recent times the requirements of regulatory authorities have become more stringent and in order that a product may be marketed it is essential to meet these new criteria. Amongst these criteria are those that the product and its ingredients be shown to be nontoxic in man and should be stable on storage under normal conditions. The toxicity of any decomposition products may be unknown or only poorly characterised. The stability of ingredients which may potentially give rise to toxic breakdown products is of particular importance in this respect, and amongst such ingredients are mercury-containing preservatives such as Thiomersal.

Liquid pharmaceutical formulations frequently contain isotonic agents to ensure that the formulation is isotonic with blood serum or lachrymal secretions. Whilst such formulations may be presented in a non-isotonic form it is generally preferred to render such formulations isotonic in order to avoid patient discomfort, biological damage to the patient or ineffective treatment. Thus, for example, non-isotonic ophthalmic formulations cause substantial stinging of the eye resulting not only in patient discomfort but in loss of the active ingredients from the site of action through watering of the eye and thus ineffective treatment.

The most commonly employed isotonic agents are ionic agents, in particular sodium chloride. For example, from FR—A—2 255 909 an isotonic ophthalmic formulation is known containing trimethoprim, polymyxin and a mercury-containing preservative like Thiomersal as well as sodium chloride as isotonic agent. However, ionic isotonic agents such as sodium chloride cause rapid decomposition of mercury-containing preservatives such as Thiomersal. The degree of decomposition is such that there is frequently none of the original preservative remaining a matter of days after the formulation is prepared, whereas the shelf-life of such formulations is measured in terms of years.

We have now surprisingly found, however, that if, for certain liquid formulations a non-ionic polyhydroxy compound is employed as an isotonic agent, then the decomposition of mercury-containing preservatives such as Thiomersal is substantially arrested, being comparable to that in simple aqueous media.

The invention accordingly provides an isotonic liquid pharmaceutical formulation, particularly an isotonic aqueous ophthalmic formulation comprising trimethoprim and polymyxin as the active ingredients, a vehicle therefor, a mercury-containing preservative and, as an isotonic agent, a non-ionic polyhydroxy compound.

The liquid formulations will be either wholly or partly aqueous in which case the non-aqueous part will be any organic solvent(s) conventionally used in such liquid formulations. The formulation will normally be a solution but other liquid formulations are within the scope of the invention. The invention is particularly applicable to injectable, otic and especially ophthalmic formulations containing trimethoprim and polymyxin.

Any suitable mercury-containing preservative may be employed, for example those described in United States Patent No. 1,672,615. The preferred preservative is Thiomersal, sodium ethylmercurithiosalicylate. Other preservatives include phenyl mercuric nitrate and phenyl mercuric acetate. The preservative should be present in an effective amount which will normally be in the range of 0.1 to 0.0001% w/v, preferably less than 0.01% w/v, particularly about 0.005% w/v.

Any non-ionic polyhydroxy containing compound (i.e. any non-ionic compound containing two or more hydroxy groups) and which is acceptable as an isotonic agent may be employed. To be acceptable as an isotonic agent, the compound will render a solution isotonic at a concentration of 10% w/v or less, preferably 5% w/v or less, will not be deleterious to the formulation and will not be deleterious or irritating to the recipient thereof. Suitable isotonic agents for use in the invention include carbohydrate compounds, particularly monomeric compounds such as dextrose, lactose, mannitol and sucrose and, preferably, polyhydric alcohols, such as propylene glycol, glycerol and low molecular weight polyethylene glycols (PEGs). By low molecular weight is meant a molecular weight of less than about 750.

Reference herein to the formulations being isotonic means for formulations being isotonic with the biological fluid with which the formulation will come into contact. The present invention is particularly concerned with ophthalmic formulations and hence the formulations will be isotonic with lachrymal secretions. Thus an isotonic solution will have an osmotic pressure of from about 270 to 320 milliosmoles per kilogram of water (mosm/kg $H_2O$), in particular about 280—300 mosm/kg $H_2O$, especially about 290 mosm/kg $H_2O$.

The isotonic agent is preferably not readily metabilised by micro-organisms. In addition it is preferred that the isotonic agent is not a nutrient for microorganisms. Thus the preferred isotonic agents

2

are polyhydric alcohols, particularly those identified above which in some cases themselves possess antimicrobial activity. The preferred isotonic agent is propylene glycol which renders conventional ophthalmic solutions isotonic with lachrymal secretions at a concentration of about 2.0% w/v.

The present invention is applicable to any isotonic liquid pharmaceutical formulation containing trimethoprim and polymyxin for example intravenous injections and otic formulations, especially when presented in a multiple dose form. However, it is particularly useful or ophthalmic formulations, such as eyedrops, which are desirably isotonic and in which a preservative is essential as the formulation is generally provided in a multiple dosage form and once opened cannot be maintained in a sterile condition.

The trimethoprim and polymyxin may be present in any form in which they are conventionally present in such formulations. In particular they may be present as acid addition salts such as sulphates. The ratio and amounts of trimethoprim to polymyxin may also be that conventionally used in such formulations i.e. from about 0.01 to 1 g trimethoprim per 1 Mega Units polymyxin, in particular about 0.1 g trimethoprim per 1 Mega Units polymyxin. The formulations conveniently contain about 1 g/litre of trimethoprim and 10 Mega Units/litre of polymyxin.

The formulations of the present invention may be prepared by any method known in the art of pharmacy for the preparation of such formulations, all of which involve the admixture of the active ingredients with the liquid vehicle.

The present invention will now be illustrated by the following examples.

Example 1
Effect of Sodium Chloride on the Stability of Thiomersal in Aqueous Solution

Thiomersal was stored as 0.1% (w/v), 0.01% (w/v) and 0.001% (w/v) solutions in water and 0.8% (w/v) Saline solution at 5°, 25° and 50°C for up to 15 days. The amount of Thiomersal remaining was assayed by high performance liquid chromatography (HPLC) after 8 and 15 days.

HPLC was carried out using a Cecil CE 210 pump with a 250 mm, 4 mm i.d. stainless steel column packed with Spherisorb 10 ODS using a mixture of methanol-water-phosphoric acid 60:50:1 as eluent at a flow rate of 2.6 ml min$^{-1}$ and a pressure of 12.5 MPa (1800 psig); injection volume 25 ul loop and operating at ambient temperature; detection by a Cecil CE 212 variable wavelength u.v. detector at 222 nm.

The results are shown in Table 1.

TABLE 1

| Thiomersal Initial | | | HPLC assay of Thiomersal % Initial concentration | |
|---|---|---|---|---|
| Concentration (per cent) | Temperature (°C) | Time (days) | water | 0.8% saline |
| 0.001 | 5 25 50 | 8 | 100.8 101.8 28.9 | 32.5 5 5 |
| 0.001 | 5 25 50 | 15 | 105.2 96.5 44.6 | 30.3 5 NA |
| 0.01 | 5 25 50 | 8 | 103.3 99.7 95.1 | 69.1 50.0 50.2 |
| 0.01 | 5 25 50 | 15 | 95.6 104.3 NA | 69.2 59.0 NA |
| 0.1 | 5 25 50 | 8 | 104.7 97.6 99.9 | 98.6 96.6 92.0 |
| 0.1 | 5 25 50 | 15 | 88.2 79.4 76.8 | 89.3 72.8 NA |

N.A. = Not Available

3

The results show that there was slight decomposition of the thiomersal in aqueous solution but that this was substantial in the presence of sodium chloride.

Example 2

Comparative effect of Isotonic Agents on Thiomersal Stability in Ophthalmic Formulations

A series of eye drop formulations containing Thiomersal (0.001% w/v) in water, trimethoprim sulphate and polymyxin sulphate (as active ingredients) and an isotonic agent were prepared. The formulations were identical with that described in Example 3 with the exception that the isotonic agents were a indicated in Table 2 below. In one case no isotonic agent was incorporated to provide a control. The amount of each isotonic agent was sufficient to render the formulations isotonic with lachrymatory secretions. The formulations were stored at 25°C for up to six weeks and the Thiomersal content determined as described in Example 1 after 3 days and 1,3 and 6 weeks.

The results are shown in Table 2.

TABLE 2

| .Isotonic Agent | Thiomersal content (% amount added) as determined by HPLC | | | | |
|---|---|---|---|---|---|
| | Initial* | 3 days | 1 week | 3 weeks | 6 weeks |
| None | 100 | — | 100 | 103 | 98 |
| Sodium Chloride | 100 | 36 | — | — | — |
| Boric Acid | 92.5 | — | 86 | 84 | 82 |
| Sodium Edetate | 101 | — | 97 | 87 | 84 |
| Propylene Glycol | 96 | — | 94.5 | 93 | 95 |
| Glycerol | 95 | — | 97.5 | 95 | 97 |
| Mannitol | 96 | — | 97.5 | 96 | 98 |

*Determined by HPLC a few hours after preparation.

The results show that the ionic isotonic agents showed a substantial loss of Thiomersal after six weeks (15—20%) whereas for the polyhydroxy compounds required by the invention losses were negligible when compared to control.

Example 3      Ophthalmic Formulation

For 100 ml

| | |
|---|---|
| Trimethoprim | 0.100 g |
| Polymyxin B Sulphate (including 10% average) | 1,1 Mega Units |
| Thiomersal | 0.005 g |
| 0.1 M Sulphuric Acid Solution | 1.722 ml |
| Propylene Glycol BP | 2.100 g |
| 0.1 M Sulphuric Acid Solution or 0.2 M Sodium Hydroxide Solution | q.s. to pH 5.0—5.2 |
| Water for injections     to produce | 100 ml |

4

Example 4

Stability of Thiomersal in Ophthalmic Formulation of the invention

Batches of the ophthalmic formulation of Example 3 were stored at 25°C for 1 year and then assayed for Thiomersal content.

The results are shown in Table 3.

TABLE 3

| Storage Time | Thiomersal Content (% amount added) | | | |
|---|---|---|---|---|
| | Sample 1 | Sample 2 | Sample 3 | Sample 4 |
| Initial | 99.4; 95.8 | 96.8; 98.2 | 95.9; 98.2 | 96.3; 95.5 |
| 1 year | 99.0 | 87.7 | 95.2 | 97.9 (after 6 months) |

## Claims

1. An isotonic liquid pharmaceutical formulation comprising trimethoprim and polymyxin, a vehicle therefor, a mercury-containing preservative and an isotonic agent characterised in that the isotonic agent comprises a non-ionic polyhydroxy compound.

2. A formulation as claimed in claim 1, in which the formulation is an ophthalmic solution.

3. A formulation as claimed in claim 1 or claim 2 wherein the isotonic agent is selected from dextrose, lactose, mannitol, sucrose, propylene glycol, glycerol and polyethylene glycols having a molecular weight of less than about 750.

4. A formulation as claimed in any one of claims 1 to 3 wherein the osmotic pressure of the formulation is from 270 to 320 mosm/kg $H_2O$.

5. A formulation as claimed in any one of claims 1 to 4 wherein the osmotic pressure of the formulation is from 280 to 300 mosm/kg $H_2O$.

6. A formulation as claimed in any one of claims 1 to 5 wherein the mercury-containing preservative is sodium ethylmercurithiosalicylate.

7. An aqueous isotonic ophthalmic formulation comprising trimethoprim (0.1 g), polymyxin B sulphate (1,1 Mega Units), sodium ethylmercurithiosalicylate (0.005 g) and propylene glycol (2.100 g) per 100 ml of formulation.

8. An isotonic liquid ophthalmic formulation comprising the following ingredients per 100 ml of final product:

| | |
|---|---|
| Trimethoprim | 0.100 g |
| Polymyxin B Sulphate | 1,1 Mega Units |
| Sodium Ethylmercurithiosalicylate | 0.005 g |
| 0.1 M Sulphuric Acid Solution | 1.722 ml |
| Propylene Glycol | 2.100 g |
| 0.1 M Sulphuric Acid Solution or 0.2 M Sodium Hydroxide Solution | q.s. to pH 5.0—5.2 |
| Water for injections | to produce 100 ml |

## Patentansprüche

1. Isotonische flüssige pharmazeutische Formulierung, enthaltend Trimethoprim und Polymyxin, einen Träger dafür, ein quecksilberhaltiges Konservierungsmittel und ein isotonisches Mittel, dadurch gekennzeichnet, daß das isotonische Mittel eine nicht-ionische Polyhydroxyverbindung enthält.

2. Formulierung nach Anspruch 1, worin die Formulierung eine ophthalmische Lösung ist.

3. Formulierung nach Anspruch 1 oder Anspruch 2, worin das isotonische Mittel unter Dextrose, Lactose, Mannit, Sucrose, Propylenglykol, Glycerin und Polyethylenglykolen mit einem Molekulargewicht von weniger als etwa 750 ausgewählt ist.

4. Formulierung nach einem der Ansprüche 1 bis 3, worin der osmotische Druck der Formulierung von 270 bis 320 mosm/kg $H_2O$ beträgt.

5. Formulierung nach einem der Ansprüche 1 bis 4, worin der osmotische Druck der Formulierung 280 bis 300 mosm/kg $H_2O$ beträgt.

6. Formulierung nach einem der Ansprüche 1 bis 5, worin das quecksilberhaltige Konservierungsmittel Natriumethylquecksilberthiosalicylat ist.

7. Wässrige isotonische ophthalmische Formulierung, welche Trimethoprim (0,1 g), Polymyxin B-Sulfat (1,1 Mega-Einheiten), Natriumethylquecksilberthiosalicylat (0,005 g) und Propylenglykol (2,100 g) pro 100 ml Formulierung enthält.

8. Isotonische flüssige ophthalmische Formulierung, welche die folgenden Bestandteile pro 100 ml des Endproduktes enthält:

| | |
|---|---|
| Trimethoprim | 0,100 g |
| Polymyxin B-Sulphat | 1,1 Mega-Einheiten |
| Natriumethylquecksilberthiosalicylat | 0,005 g |
| 0,1 M Schwefelsäure-Lösung | 1,722 ml |
| Propylenglykol | 2,100 g |
| 0,1 M Schwefelsäure-Lösung oder 0,2 M Natriumhydroxid-Lösung | q.s. auf pH 5,0 bis 5,2 |
| Wasser für Injektionen zur Herstellung von | 100 ml. |

## Revendications

1. Formulation pharmaceutique liquide isotonique comprenant du trimethoprim et de la polymyxine, un véhicule pour ceux-ci, un agent de conservation contenant du mercure et un agent isotonique, caractérisée en ce que l'agent isotonique comprend un composé polyhydroxylé non ionique.

2. Formulation suivant la revendication 1, qui est une solution ophtalmique.

3. Formulation suivant la revendication 1 ou 2, dans laquelle l'agent isotonique est choisi parmi le dextrose, le lactose, le mannitol, le saccharose, le propylèneglycol, le glycérol et les polyéthylèneglycols d'un poids moléculaire inférieur à environ 750.

4. Formulation suivant l'une quelconque des revendications 1 à 3, dans laquelle la pression osmotique de la formulation est de 270 à 320 mosm/kg $H_2O$.

5. Formulation suivant l'une quelconque des revendications 1 à 4, dans laquelle la pression osmotique de la formulation est de 280 à 300 mosm/kg $H_2O$.

6. Formulation suivant l'une quelconque des revendications 1 à 5, dans laquelle l'agent de conservation contenant du mercure est l'éthylmercurithiosalicylate de sodium.

7. Formulation ophtalmique isotonique aqueuse comprenant du triméthoprim (0,1 g), du sulfate de polymyxine B (1,1 méga-unité), de l'éthylmercurithiosalicylate de sodium (0,005 g) et du propylèneglycol (2,100 g) pour 100 ml de formulation.

8. Formulation ophtalmique liquide isotonique comprenant les constituants suivants pour 100 ml de produit final:

| | |
|---|---|
| Triméthoprim | 0,100 g |
| Sulfate de polymyxine B | 1,1 méga-unité |
| Ethylmercurithiosalicylate de sodium | 0,005 g |
| Solution d'acide sulfurique 0,1 M | 1,722 ml |
| Propylèneglycol | 2,100 g |
| Solution d'acide sulfurique 0,1 M<br>ou<br>solution d'hydroxyde de sodium 0,2 M } | q.s. pour pH 5,0—5,2 |
| Eau pour injections | pour 100 ml |